# EUROPÄISCHE PATENTSCHRIFT

(11) **EP 2 153 818 B1**
(45) Veröffentlichungstag und Bekanntmachung des Hinweises auf die Patenterteilung: **05.09.2018**
(21) Anmeldenummer: 09164364.3
(22) Anmeldetag: 02.07.2009
(51) Int. Cl.: A61Q 5/12, A61K 8/898

(54) **Verwendung von Polysiloxanen mit quaternären Ammoniumgruppen zum Schutz von tierischen oder menschlichen Haaren vor Hitzeschädigung**
Use of polysiloxanes with quarternary ammonium groups for protecting animal or human hair from heat damage
Utilisation de polysiloxanes dotés de groupes d'ammonium quaternaires pour la protection de poils d'animaux ou d'humains contre les dommages dus à la chaleur

(30) Priorität: 06.08.2008 DE 102008041020
(43) Veröffentlichungstag der Anmeldung: 17.02.2010
(73) Patentinhaber: Evonik Degussa GmbH, 45128 Essen (DE)
(72) Erfinder: Herrwerth, Sascha, 45134 Essen (DE); Ferenz, Michael, 45147 Essen (DE); Winter, Patrick, 45473 Mülheim an der Ruhr (DE)

(56) Entgegenhaltungen:
- EP-A1- 1 810 657
- EP-A1- 1 887 024
- EP-A2- 0 294 642
- WO-A1-2006/040286
- EVONIK INDUSTRIES: "ABIL TM T Quat 60. Premium conditioning agent with additional hair fiber protection properties", 20080201, 1. Februar 2008 (2008-02-01), Seiten 1-5, XP007913393,
- NO AUTHOR NAME SUPPLIED IN SOURCE DATA: "THERMAL PROTECTION BENEFITS ON HAIR FROM SILICONE CONDITIONING POLYMERS", IP.COM JOURNAL, IP.COM INC., WEST HENRIETTA, NY, US, 14. September 2004 (2004-09-14), XP013021440, ISSN: 1533-0001
- Trefor A. Evans ET AL: "Hair testing - quantifying the benefits of conditioning treatments on hair breakage", , 9. Dezember 2010 (2010-12-09), Seiten 1-4, XP55000533, [gefunden am 2011-06-10]
- "ABIL T Quat 60 (structure)", , 9. Dezember 2010 (2010-12-09), XP55000539, [gefunden am 2011-06-10]
- "Silsoft Tone conditioning agent with thermal protection", , 1. Mai 2009 (2009-05-01), Seiten 1-8, XP55000298, Gefunden im Internet: URL:http://www.momentive.com/momentiveInte rnetDoc/Internet/Static%20Files/Documents/ Marketing%20Bulletin/Silsoft%20Tone%20with %20Thermal%20Protection%20MB.indd.pdf [gefunden am 2011-06-08]
- "International Cosmetic Ingredient and Dictionary Handbook", 2008, The Cosmetic, Toiletry, and Fragrance Association vol. 1, page 164, * entry "Amodimethicone" *
- TODD OSTERGAARD ET AL: "Silicone Quaternary Microemulsion: A Multifunctional Product for Hair Care", COSMETICS & TOILETRIES, WHEATON, IL, US, vol. 119, no. 11, 1 November 2004 (2004-11-01), pages 45-48,50,52, XP009125007, ISSN: 0361-4387
- DOW CORNING: "DOW CORNING TM 5-7070 Si Amino Elastomer Emulsion", PRODUCT INFORMATION PERSONAL CARE DOW CORNING,, 15 July 2008 (2008-07-15), pages 1-6, XP007910347,
- "Dow Corning 5-7070 Si Amino Elastomer Emulsion Offers Styling and Conditioning Benefits for Hair Care Applications - SpecialChem", , 6 July 2007 (2007-07-06), XP0055210121, Retrieved from the Internet: URL:http://cosmetics.specialchem.com/news/ product-news/dow-corning-5-7070-si-amino-e lastomer-emulsion-offers-styling-and-condi tioning-benefits-for-hair-2074 [retrieved on 2015-08-28]

## Beschreibung

### Gebiet der Erfindung

Gegenstand der Erfindung ist die Verwendung von Polysiloxanen beinhaltend mindestens eine quaternäre Ammoniumgruppe zum Schutz von tierischen oder menschlichen Haaren vor Hitzeschädigung.

### Stand der Technik

Menschliches Haar ist täglich den verschiedensten Einflüssen ausgesetzt. Neben mechanischen Beanspruchungen durch Bürsten, Kämmen, Hochstecken oder Zurückbinden werden die Haare auch durch Umwelteinflüsse wie zum Beispiel starke UV-Strahlung, Kälte, Hitze, Wind und Wasser angegriffen. Auch der physiologische Status (z.B. Alter, Gesundheit) der jeweiligen Person beeinflusst den Zustand der keratinischen Fasern.

Insbesondere die Behandlung mit chemischen Mitteln verändert Struktur und Oberflächeneigenschaften der Haare. Methoden wie zum Beispiel das Dauerwellen, Bleichen, Färben, Tönen, Glätten usw., aber auch häufiges Waschen mit aggressiven Tensiden tragen dazu bei, dass mehr oder weniger starke Schäden an der Haarstruktur verursacht werden. So wird zum Beispiel bei einer Dauerwelle sowohl der Cortex als auch die Cuticula des Haares angegriffen. Die Disulfid-Brücken des Cystins werden durch den Reduktionsschritt aufgebrochen und im anschließenden Oxidationsschritt zum Teil zu Cysteinsäure oxidiert.

Die Bandlung der Kopfhaare zur Reinigung, Färbung, Wellung, Glättung, Festigung oder Formung wird überwiegend mit wässrigen Zubereitungen durchgeführt. In Anschluss an diese Behandlungsverfahren soll das Haar wieder getrocknet werden. Um dies in kurzer Zeit zu erzielen, wird die Trocknung unter Verwendung von Heißluft durchgeführt. Die Verwendung von Heißluft-Föns dient aber auch einer Verstärkung der formgebenden und der festigenden Behandlung. Auch Lockenstäbe werden mit dem Ziel der dauerhaften Fixierung einer Haarkräuselung eingesetzt.

Des Weiteren werden Glätteisen zur Glättung und Fixierung von Haaren verwendet, die Temperaturen größer 200 °C erzeugen können.

Durch die genannten Verfahren der Heißluft- und Hitzeanwendungen zur Trocknung und Formgebung werden die Haare in ihrer Struktur geschädigt.

Man hat dem Problem der Haarschädigung durch Hitzeeinwirkung schon seit langem Aufmerksamkeit gewidmet, und es wurden schon zahlreiche Vorschläge gemacht, durch Zusätze zu Haarbehandlungsmitteln das Problem der thermischen Haarschädigung zu mindern.

Die WO 00/44337 offenbart die Verwendung von Copolymeren aus Polysiloxan und Proteinen in kosmetischen Formulierungen zum Schutz von Haaren vor Schädigungen durch Hitzebehandlungen. Ähnlich Strukturen werden auch in dem Artikel "Test method for the study of ingredients protecting hair against damage caused by thermal stresses" von S. Meulemann (SÖFW-Journal128. Jahrgang, 3, 2008) beschrieben.

In der US 6241977 werden Copolymere aus Vinylether und Maleinsäure als Wirkstoffe beschrieben, die Haare und Wolle vor Schädigung durch Hitze im Bereich von 100 - 180 °C schützen.

In der DE 19943597 wird die Verwendung von basischen Aminosäuren in wäßrigen Haarbehandlungsmitteln als Fönschutz des Haares offenbart. Bevorzugt geeignet ist L-Histidin in solchen Formulierungen, die der Formung und Festigung der Haare unter Verwendung von Heißluft dienen.

In JP 03157316 wird z.B. vorgeschlagen, eine Kombination aus quaternären Ammoniumsalzen, bestimmten Pflanzenextrakten und wasserlöslichen Polymeren mit quaternären Ammoniumgruppen einzusetzen.

In JP 03135909 wird vorgeschlagen, hochmolekulare Silikone und Fettsäurealkanolamide einzusetzen.

Gemäß WO 99/11224 eignet sich eine Kombination aus einem faserstrukturverbessernden Wirkstoff wie z.B. Panthenol und einem konditionierenden Wirkstoff wie z.B. einem kationischen Tensid, um die Hitzeschädigung des Haares zu vermindern.

EP 1 810 657 A1 offenbart die Verwendung kationischer Silikone zum Schutz von Haaren vor Hitzeschädigung. Es besteht daher also weiterhin ein Bedarf an vielseitig einsetzbaren Wirkstoffen für reinigende und pflegende Formulierungen für Haare wie z.B. Shampoos, Leave-In Conditioner, Haarspülungen und Haarnachbehandlungsmittel, die neben der reinigenden und pflegenden Wirkung die Haares effektiv vor der Schädigung durch Hitzeeinwirkung schützen, und die Veränderung der Haarstruktur minimieren.

Polysiloxane mit quaternären Gruppen und deren Anwendung als Additive für Haarpflege oder Textilweichmacher sind aus der Patentliteratur bekannt.

So werden zum Beispiel in der DE 14 93 384, EP 0017122 und US 4895964 Strukturen beschrieben, bei denen Siloxane mittelständig mit statistisch über das Polymer verteilten Ammoniumgruppen modifiziert sind. Diese Verbindungen haben den Nachteil, dass ein ausgeprägter Siliconcharakter vermindert wird und eine gute Wirksamkeit nicht mehr zu beobachten ist.

Einen ausgeprägteren Siliconecharakter haben kationische Polysiloxane, wie sie in DE 37 19 086 und EP 0 294 642 beschrieben werden. Die in der DE 37 19 086 und die in der EP 0 294 642 beschreiben Strukturen, bei denen die quaternären Funktionen endständig an das Polysiloxan gebunden sind. Derartige Verbindungen bieten Vorteile hinsichtlich ihrer Wirkung als Konditioniermittel sowohl für Haare und Textilien als auch für harte Oberflächen. Die Verwendung solcher Verbindungen in kosmetischen Formulierungen ist z.B. in der EP 0 530 974, EP 617 607, EP 1080714, WO 2001082879 und US 6207141 beschrieben worden.

In der EP 1887024 werden endständig kationische Polysiloxane mit einer T-Struktur und deren Verwendung als Konditioniermittel in kosmetischen Formulierungen beschrieben. Diese kationischen Polysiloxane zeigen einen ausgeprägten konditionierenden und glanzerzeugenden Effekt.

In keiner der genannten Schriften wird ein Einfluss der Polysiloxane beinhaltend quaternäre Ammoniumgruppen auf die Schädigung beschrieben, die das Haar durch Hitzeeinwirkung erleidet.

Aufgabe der Erfindung war es, einen Wirkstoff zur Verfügung zu stellen, der in der Lage ist, das Haar signifikant vor der Schädigung durch Hitzeeinwirkung zu schützen.

### Beschreibung der Erfindung

Auf der Suche nach Wirkstoffen, die das Haar vor Hitzeschädigung schützen, wurde überraschend festgestellt, dass kationische Polysiloxane eine signifikante Schutzwirkung vor Hitzeeinwirkung für das Haarkeratin entfalten.

Gegenstand der Erfindung ist daher die Verwendung von Polysiloxanen beinhaltend mindestens eine quaternäre Ammoniumgruppe nach Ansppruch 1 zum Schutz von tierischen oder menschlichen Haaren vor Hitzeschädigung.

Ein Vorteil der erfindungsgemäßen Verwendung ist, dass diese Polysiloxane zu einem verbesserten Anschäumverhalten, einem erhöhten Schaumvolumen und einer besseren Schaumcremigkeit der Formulierungen beitragen.

Ein weiterer Vorteil der erfindungsgemäßen Verwendung ist, dass die Polysiloxane mit quaternären Funktionen hervorragende konditionierende Effekte auf Haut und Haar ausüben können. Durch diesen konditionierenden Effekt auf beispielsweise der Haut kann einem trockenen, spröden oder rauen Zustand der Haut nach Anwendungen einer wässrigen, tensidischen Formulierung vorgebeugt werden und ein angenehmes, samtig-seidiges Hautgefühl erzielt wird.

Noch ein weiterer Vorteil der erfindungsgemäßen Verwendung ist, dass Eigenschaften wie Kämmbarkeit, Weichheit, Volumen, Formbarkeit, Glanz, Handhabbarkeit und die Entwirrbarkeit von ungeschädigten und geschädigten Haaren verbessert wird.

Der Begriff "quaternäre Ammoniumgruppe" im Sinne der vorliegenden Erfindung beinhaltet eine einwertig positiv geladene Gruppe mit einem vierbindigen Stickstoff.

Als Beispiel sei folgende allgemeine Formel genannt 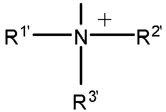 wobei R^{1'}, R^{2'} und R^{3'} unabhängig voneinander gleiche oder ungleiche, verzweigte oder unverzweigte, gegebenenfalls substituierte Kohlenwasserstoffreste, die gegebenenfalls Ester-, Amid- oder Etherfunktionen einhalten, darstellt.

Der Begriff "endständig an das Polysiloxan gebunden" im Sinne der vorliegenden Erfindung beinhaltet, dass die quaternäre Ammoniumgruppe an das Siloxangerüst über eine sogenannte M'-Einheit der allgemeinen Formel XSiY₂O_{1/2} gebunden ist. X und Y sind wie unten angegeben definiert (siehe Formel 1).

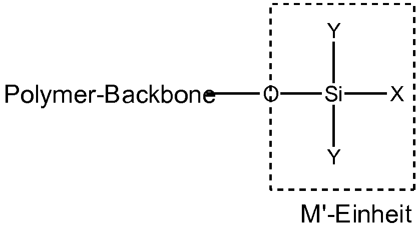

Der Begriff "wasserunlöslich" im Sinne der vorliegenden Erfindung ist definiert als eine Löslichkeit von weniger als 0.01 Gewichtsprozent in wässriger Lösung bei 20 °C und 1 bar Druck.

Der Begriff "wasserlöslich" im Sinne der vorliegenden Erfindung ist definiert als eine Löslichkeit von gleich oder mehr als 0.01 Gewichtsprozent in wässriger Lösung bei 20 °C und 1 bar Druck.

Werden im Rahmen der vorliegenden Erfindung Verbindungen, wie z. B. Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen.

Alle angegebenen Prozent (%) sind, wenn nicht anders angegeben, Massenprozent.

Alle Bedingungen wie beispielsweise Druck und Temperatur sind, wenn nicht anders angegeben, Standardbedingungen.

Die Hitzeschädigungen, vor denen geschützt werden sollen, können durch sämtliche Wärmequellen hervorgerufen werden.

Die auf das Haar treffende Hitze kann beispielsweise direkt oder durch Strahlung oder durch Konvektion auf das Haar einwirken. Beispiele für alltägliche, auf Haar einwirkende Hitze verursachende Geräte umfassen: z.B. Heißluft-Föns, Glätteisen und Lockenstäbe.

Die Hitze kann auch natürlichen Ursprungs z.B. Sonneneinstrahlung sein.

Bevorzugt handelt es sich bei den erfindungsgemäß verwendeten Polysiloxane um solche, deren Gesamtladung positiv ist und somit um kationische Polysiloxane.

Es können wasserlösliche oder wasserunlösliche Polysiloxane verwendet werden. Je nach zu erzeugender Formulierung (trübe oder klare Formulierungen) ist es dem Fachmann geläufig, ob wasserlösliche oder unlösliche Polysiloxane zur Herstellung der Formulierung eingesetzt werden sollten.

Bevorzugt beinhaltet das erfindungsgemäß verwendete Polysiloxan mindestens zwei, besonders bevorzugt mindestens drei quaternäre Ammoniumgruppen.

Erfindungsgemäß verwendet werden bevorzugt Polysiloxane, bei denen mindestens eine quaternäre Ammoniumgruppe endständig an das Polysiloxan gebunden ist, besonders bevorzugt beinhalten verwendete Polysiloxane insgesamt genau zwei und endständig an das Polysiloxan gebundene quaternäre Ammoniumgruppen. Beispiele für solche Polysiloxane sind der DE 37 19 086 und der EP 0 294 642 zu entnehmen.

Darüber hinaus ist es ganz besonders bevorzugt, Polysiloxane der allgemeinen Fromel I erfindungsgemäß zu verwenden.

[M' Dₙ]₃ T Formel I

Dabei sind:
- M': = XSiY₂O_{1/2}
- D: = SiY₂O_{2/2}
- T: = SiZO_{3/2}
- X: sind gleiche oder verschiedene organische Reste, die quaternäre Ammoniumgruppen tragen,
- Y: sind gleiche oder verschiedene Reste aus der Gruppe Alkyl, Aryl, oder Alkaryl mit 1 bis 30 C-Atomen, vorzugsweise Methyl oder Phenyl, insbesondere Methyl,
- Z: sind gleiche oder verschiedene Reste aus der Gruppe Alkyl, Aryl, oder Alkaryl mit 1 bis 30 C-Atomen, vorzugsweise Methyl oder Phenyl,
- n: ist 2 bis 200, bevorzugt 3 bis 120, insbesondere 8 bis 80.

Geeignete Reste X sind zum Beispiel Gruppen mit dem Aufbau -R1-R2, worin
- R1: vorzugsweise gleiche oder verschiedene zweiwertige Reste sind, ausgewählt aus der Gruppe 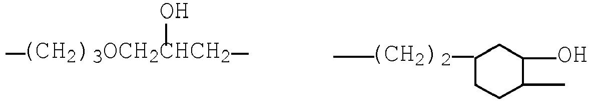 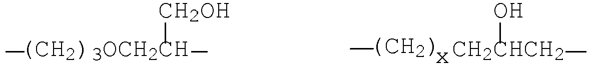 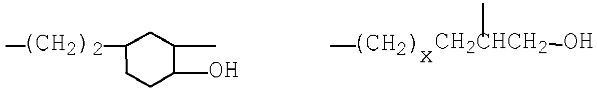 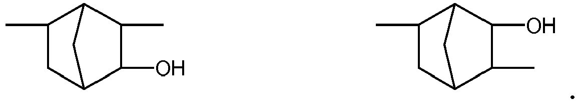 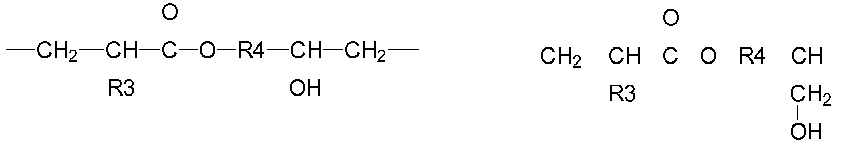
- R1: ist bevorzugt: 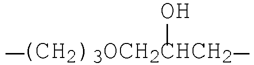
- R2: ausgewählt ist aus der Gruppe bestehend aus 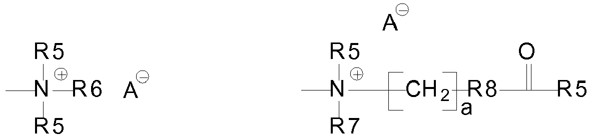 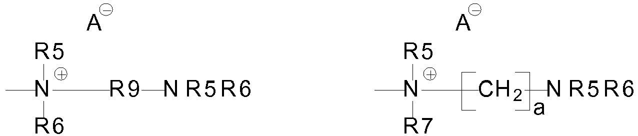 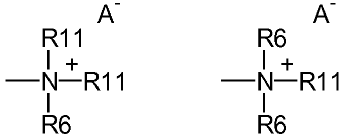
- R3: sind gleiche oder verschiedene Reste aus der Gruppe Wasserstoff oder Alkyl mit 1 bis 6 C-Atomen, bevorzugt Methyl,
- R4: sind gleiche oder verschiedene zweiwertige Kohlenwasserstoffreste, die gegebenenfalls Etherfunktionen enthalten, bevorzugt Methylen,
- R5, R6, R7: sind jeweils unabhängig voneinander Wasserstoff oder Alkylreste mit 1 bis 30 C-Atomen,
- R8: sind gleiche oder verschiedene Reste aus der Gruppe -O-; -NR10,
- R9: sind gleiche oder verschiedene gegebenenfalls verzweigte divalente Kohlenwasserstoffreste,
- R10: sind gleiche oder verschiedene Reste aus der Gruppe Wasserstoff oder Alkyl mit 1 bis 6 C-Atomen,
- R11: sind gleiche oder verschiedene Reste der allgemeinen Formel: 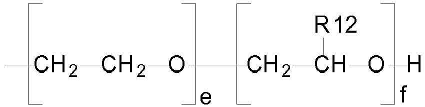
- R12: sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl,
- e: 0 bis 20, bevorzugt 0 bis 10, insbesondere 1 bis 3,
- f: 0 bis 20, bevorzugt 0 bis 10,
- e + f: >= 1,
- x: 2 bis 18,
- a: 2 bis 18, vorzugsweise 3,
- A⁻: sind gleiche oder verschiedene Gegenionen zu den positiven Ladungen an den quaternären Ammoniumgruppen, ausgewählt aus anorganischen oder organischen Anionen der Säuren HA.

Beispiele für solche Polysiloxane sind der EP 1887024 beschrieben.

Ein Vorteil der Verwendung der Polysiloxanen gemäß Formel I ist ein reduzierter viskositätserniedrigender Effekt im Vergleich zu den kationischen Polysiloxanen, wie diese in DE 37 19 086 und EP 0 294 642 beschrieben werden. Das führt zu einer signifikanten Reduktion an benötigten Verdicker, um die Formulierungen auf die gewünschte Viskosität einzustellen. Dadurch wird eine Vereinfachung der Formulierung ermöglicht, die dem Ressourcen schonenden Aspekt Rechnung trägt.

Die erfindungsgemäße Verwendung der Polysiloxane findet normalerweise in Form von Applikation von kosmetischen Formulierungen statt.

Bevorzugt handelt es sich bei den kosmetischen Formulierungen um reinigende und pflegende Formulierungen. Unter reinigende und pflegende Formulierungen werden vor allem solche Formulierungen zur Behandlung der Haare verstanden, nach deren Anwendung das Haar üblicherweise mit Heißluft-Föns oder Glätteisen behandelt wird. Solche Haarbehandlungsmittel sind z.B. Haarshampoos, Flüssigseifen, Haarspülungen, Dauerwell-Fixierlösungen, Haarfärbeshampoos, Haarfestiger, Haarlegemittel, Haarstyling-Zubereitungen, Fön-Lotionen, Schaumfestiger, Haarkuren, Leave-In Conditionierer und andere reinigende und pflegende Formulierungen.

Bei der Herstellung der kosmetischen Formulierungen nach Anspruch 7 zum Schutz von menschlichem oder tierischem Haar vor Hitzeschädigung werden die oben beschriebenen, vorzugsweise die oben als bevorzugt beschriebenen Gruppen von Polysiloxanen verwendet.

Die Polysiloxane beinhaltend mindestens eine quaternäre Ammoniumgruppen werden in einer Konzentration von 0.01 bis 20 Massenprozent, bevorzugt 0.1 bis 8 Massenprozent, besonders bevorzugt von 0.2 bis 4 Massenprozent in den kosmetischen Formulierungen nach Anspruch 7 verwendet.

Ein weiterer Gegenstand der vorliegenden Erfindung sind somit auch die kosmetischen Formulierungen zum Schutz von menschlichem oder tierischem Haar vor Hitzeschädigung nach Anspruch 7, die durch die Verwendung der Polysiloxane beinhaltend mindestens eine quaternäre Ammoniumgruppe zur Herstellung der Formulierungen erhalten werden.

Die erfindungsgemäßen kosmetischen Formulierungen zum Schutz von menschlichem oder tierischem Haar vor Hitzeschädigung werden vorzugsweise erhalten durch die Verwendung der oben beschriebenen, insbesondere der oben als bevorzugt beschriebenen Gruppen von Polysiloxanen zur Herstellung der Formulierungen.

Die kosmetischen Formulierungen zum Schutz von menschlichen oder tierischen Haaren vor Hitzeschädigung enthaltend Polysiloxane enthaltend quaternäre Ammoniumgruppen können z.B. mindestens eine zusätzliche Komponenten enthalten, ausgewählt aus der Gruppe der
Emollients,
Emulgatoren und Tenside,
Verdicker/Viskositätsregler/Stabilisatoren,
Antioxidantien und Vitamine,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Biogene Wirkstoffe,
Pflegeadditive,
Überfettungsmittel
Lösungsmittel.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyl-adipat, Di-n-butylsebacat, Di-(2-ethylhexyl)-adipat, Di-(2-hexyldecyl)-succinat, Di-isotridecylacelaat. Geeignete Diolester sind z.B. Ethylenglycoldioleat, Ethylenglycol-di-isotridecanoat, Propylenglycol-di-(2-ethylhexanoat), Butandiol-diisostearat, Butandiol-di-caprylat/caprat und Neopentylglycol-di-caprylat. Weitere Fettsäureester, die als Emollients eingesetzt werden können, sind z.B. C₁₂-₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen C6- C22-Fettalkoholen, Ester von verzweigten C6-C13-Carbonsäuren mit linearen C6-C22-Fettalkoholen, Ester von linearen C6-C22-Fettsäuren mit verzweigten C8-C18-Alkoholen, insbesondere 2-Ethylhexanol oder Isononanol, Ester von verzweigten C6-C13-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C6-C22-Fettalkoholcarbonate, Guerbetcarbonate, Ester der Benzoesäure mit linearen und/oder verzweigten C6-C22-Alkoholen (z. B. Finsolv™ TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als Emulgatoren oder Tenside können nichtionische, anionische, kationische oder amphotere Tenside eingesetzt werden.

Als nichtionogene Emulgatoren oder Tenside können Verbindungen aus mindestens einer der folgenden Gruppen eingesetzt werden:
Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
C_{12/18}-Fettsäuremono- und -diester von Anlagerungsprodukten von 1 bis 100 Mol Ethylenoxid an Glycerin,
Glycerinmono- und -diester und Sorbitanmono- und -diester von gesättigten und ungesättigten Fettsäuren mit 6 bis 22 Kohlenstoffatomen und deren Ethylenoxidanlagerungsprodukte,
Alkylmono- und -oligoglycoside mit 8 bis 22 Kohlenstoffatomen im Alkylrest und deren Ethylenoxidanlagerungsprodukte,
Anlagerungsprodukte von 2 bis 200 Mol Ethylenoxid an Ricinusöl und/oder gehärtetes Ricinusöl,
Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15,
Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL® EM 90 (Evonik Goldschmidt GmbH)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z.B. Glycerin oder Polyglycerin,
Zitronensäureester wie z.B. Glyceryl Stearate Citrate, Glyceryl Oleate Citrate und Dilauryl Citrate.

Anionische Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid.

Weiterhin können Monoalklyamidoquats wie z.B. Palmitamidopropyltrimethylammoniumchlorid oder entsprechende Dialkylamidoquats eingesetzt werden.

Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.

Typische Beispiele für milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen.

Weiterhin ist es möglich, amphotere Tenside wie z.B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Von den ampholytischen Tensiden können solche oberflächenaktiven Verbindungen eingesetzt werden, die außer einer C8/18-Alkyl- oder -Acylgruppe im Molekül mindestens eine freie Aminogruppe und mindestens eine-COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C12/18-Acylsarcosin.

Geeignete Verdicker sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z. B. Carbopole TM oder Synthalene TM), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und/oder Metallsalze von Fettsäuren, wie z.B. Zinkstearat eingesetzt werden.

Als Viskositätsregler für wässrige Tensidsysteme können z.B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:
3-Benzylidencampher und dessen Derivate, z.B. 3-(4-Methylbenzyliden) campher,
4-Aminobenzoesäurederivate, wie z.B. 4-(Dimethylamino)benzoesäure-2-ethylhexylester,4-(Dimethylamino)benzoesäure-2-ethylhexylester und 4-(Dimethylamino)benzoesäureamylester Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene),
Ester der Salicylsäure, wie z.B. Salicylsäure-2-ethylhexylester, Salicylsäure-4-isopropylbenzylester, Salicylsäurehomomenthylester,
Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon, Ester der Benzalmalonsäure, wie z.B. 4-Methoxybenzmalonsäuredi-2-ethylhexyester,
Triazinderivate, wie z.B. 2,4,6-Trianilino-(p-carbo-2'-ethyl-1'-hexyloxy)-1,3,5-triazin und Octyltriazon,
Propan-1,3-dione, wie z.B. 1-(4-tert.Butylphenyl)-3-(4'-methoxyphenyl)propan-1,3-dion.
Als wasserlösliche UVB-Lichtschutzfilter kommen in Frage: 2-Phenylbenzimidazol-5-sulfonsäure und deren Alkali-, Erdalkali-, Ammonium-, Alkylammonium-, Alkanolammonium- und Glucammoniumsalze,
Sulfonsäurederivate von Benzophenon, wie z.B. 2-Hydroxy-4-methoxybenzophenon-5-sulfonsäure und ihre Salze,
Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen. Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Als Antioxidantien und Vitamine können z.B. Superoxid-Dismutase, Tocopherol (Vitamin E), Tocopherolsorbat, Tocopherolacetat, andere Ester von Tocopherol, Dibutylhydroxytoluol und Ascorbinsäure (Vitamin C) und ihre Salze sowie deren Derivate (z.B. Magnesium Ascorbylphosphat, Natrium Ascorbylphosphat, Ascorbylsorbat), Ascorbyl Ester von Fettsäuren, butylierte Hydroxybenzoesäure und ihre Salze, Peroxide wie z.B. Wasserstoffperoxid, Perborate, Thioglycolate, Persulfatsalze, 6-Hydroxy-2,5,7,8-Tetramethylchroman-2-Carboxylsäure (TROLOX.RTM), Gallussäure und ihre Alkylester, Harnsäure und ihre Salze und Alkylester, Sorbinsäure und ihre Salze, Liponsäure, Ferulasäure, Amine (z.B. N,N-Diethylhydroxylamin, Amino-Guanidine), Sulfhydryl-Verbindungen (z.B. Glutathion), Dihydroxy-Fumarsäure und ihre Salze, Glycinpidolat, Argininpilolat, Nordihydroguaiaretissche Säure, Bioflavonoide, Curcumin, Lysin, L-Methionin, Prolin, Superoxid Dismutase, Silymarin, Tee Extract, Grapefruit Schalen/Kern Extrakt, Melanin, Rosmarin Extrakt, Thioctansäure, Resveratrol, Oxyresveratrol etc. eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:
Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere. Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filter- noch färbende Wirkung haben, beispielsweise Aerosile® (CAS-Nr. 7631-86-9).

Als Filmbildner zur z.B. Verbesserung der Wasserfestigkeit können beispielsweise eingesetzt werden: Polyurethane, Dimethicone, Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z.B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Illite, Beidelit, Nontronit, Saponit, Hectorit, Bentonit, Smectit, ferner beispielsweise Zinksalze der Ricinolsäure in Frage. Keimhemmende Mittel sind ebenfalls geeignet, eingearbeitet zu werden. Keimhemmende Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO® Cosmo P813, Degussa), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methlyparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethyl-isothiazolin, Ethylhexylglycerin oder Caprylyl Glycol eingesetzt werden. Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid,
Palmit amidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Polyphenole, Desoxyribonucleinsäure, Coenzym Q10, Retinol, AHA-Säuren, Aminosäuren, Hyaluronsäure, alpha-Hydroxysäuren, Isoflavone, Polyglutaminsäure, Creatin (und Creatinderivate), Guanidin (und Guanidinderivate), Pseudoceramide, essentielle Öle, Peptide, Proteinhydrolysate, Pflanzenextrakte, Bisabolol, Allantoin, Panthenol, Phytantriol, Idebenon, Lakritz Extrakt, Glycyrrhizidin und Idebenon, Skleroglucan, β-Glucan, Santalbinsäure und Vitaminkomplexe zu verstehen. Beispiele für Pflanzenextrakte sind Kastanien Extrakt, Kamillen Extrakt, Rosmarin Extrakt, schwarze und rote Johannisbeer Extrakt, Birken Extrakt, Hagebutten Extrakt, Algen Extrakte, Grüner Tee Extrakt, Aloe Extrakt, Ginseng Extrakt, Ginko Extrakt, Grapefruit Extrakt, Calendula Extrakt, Kampher, Thymus Extrakt, Mangosteen Extrakt, Cystus Extrakt, Terminalia Arjuna Extrakt, Hafer Extrakt, Oregano Extrakt, Himbeer Extrakt, Erdbeer Extrakt, etc.

Zu den biogenen Wirkstoffen können auch die sogenannten Barriere Lipids gezählt werden, für welche beispielhaft Ceramide, Phytosphingosin und Derivate, Sphingosin und Derivate, Sphinganin und Derivate, Pseudoceramide, Phospholipide, Lysophospholipide, Cholesterin und Derivate, Cholesteryl Ester, freie Fettsäuren, Lanolin und Derivate, Squalan, Squalen und verwandte Substanzen genannt werden.

Zu den biogenen Wirkstoffen werden im Sinne der Erfindung auch anti-Akne wie z.B. Benzylperoxid, Phytosphingosin und Derrivate, Niacinamid Hydroxybenzoat, Nicotinaldehyd, Retinolsäure und Derrivate, Salicylsäure und Derrivate, Citronellsäure etc. und anti-Cellulite wie z.B. Xanthin Verbindungen wie Coffein, Theophyllin, Theobromin und Aminophyllin, Carnitin, Carnosin, Salicyloyl Phytosphingosin, Phytosphingosine, Santalbinsäure etc. gezählt, ebenso wie Antischuppenmittel wie beispielsweise Salicylsäure und Derrivate, Zink Pyrithion, Selensulfid, Schwefel, Ciclopiroxolamin, Bifonazol, Climbazol, Octopirox und Actirox etc, ebenso wie Adstringetien wie z.B. Alkohol, Aluminium Derivate, Gallsäure, Pyridoxinsalicylat, Zinksalze wie z.B. Zinksulfat, -acetat, -chlorid, -lactat, Zirconiumchlorohydrate etc. Ebenso können Bleichmittel wie Kojisäure, Arbutin, Vitamin C und Derivate, Hydroquinon, Turmeric oil, Creatinin, Sphingolipide, Niacinamid etc. zu den biogenen Wirkstoffen gezählt werden.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z.B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Figuren sind Bestandteil der Beispiele:

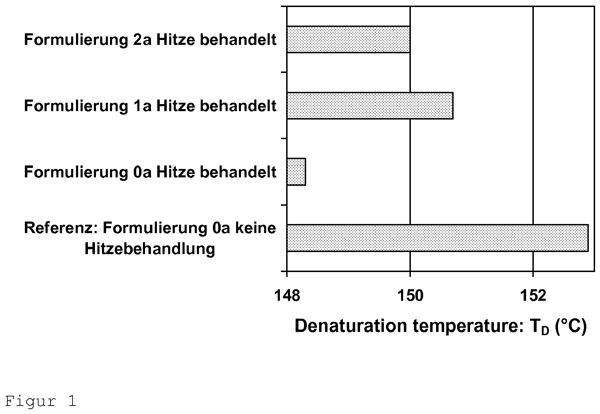

Figur 1: DSC-Messergebnisse.

### Beispiele:

### Beispiel 1: Herstellung von einem kationischen Polysiloxan mit endständigen quaternären Funktionen:

### a) Equilibrierung eines SiH-funktionellen Polysiloxans

In einem 500 mL Dreihalskolben wurden 16,4 g Phenyltris(dimethylsiloxy)silan, 232 g Decamethylpentasiloxan, 0,25 g eines sauren Katalysators gemischt und 4 Stunden bei 80 °C gerührt. Nach dem Abkühlen wurden 20 g NaHCO₃ zugegeben und bei Raumtemperatur 12 Stunden gerührt. Nach einer Filtration wurde ein klares Produkt erhalten.

### b) Herstellung eines Epoxysiloxans

In einem 500 mL Dreihalskolben wurden 300 g der gemäß 1 a) hergestellten Verbindung und 9,3 g Allylglycidylether zusammen vorgelegt und auf 100 °C aufgeheizt. Anschließend wurden 15 ppm eines Platin-Katalysators zugesetzt und zwei Stunden lang gerührt. Nach einer anschließenden Reaktion erhielt man ein klares Produkt mit einem Epoxywert von 0,94 %.

### c) Umsetzung zum quaternären Polysiloxan-Polymer

In einem 500 mL Dreihalskolben wurden 36 g 3-N,N-Dimethyl-aminopropyl-laurylsäureamid, 11 g Milchsäure und 100 g Isopropanol bei Raumtemperatur gerührt. Anschließend wurden 200 g der gemäß 1 b) hergestellten Verbindung zugetropft. Anschließend wurde 8 Stunden bei 50 °C gerührt und destilliert. Es wurde eine trübe hochviskose Flüssigkeit erhalten, welches durch die folgende statistische Formel beschrieben wird: 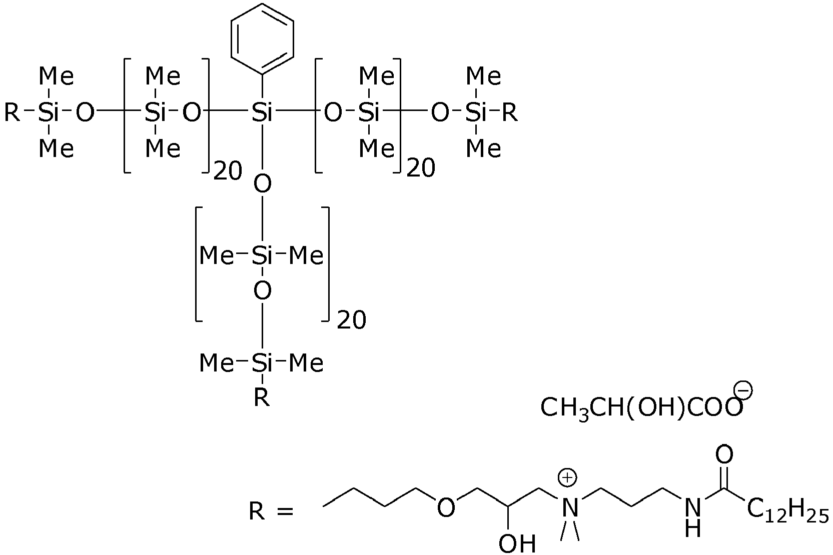 Dem Fachmann ist geläufig, dass die oben angegebene Formel eine idealisierte Strukturformel darstellt. Im Produkt liegen zusätzlich lineare und höher verzweigte Strukturen vor.

### Beispiel 2: Herstellung von einem linearen kationischen

*Polysiloxan mit zwei endständigen quaternären Funktionen:*

### a) Equilibrierung eines SiH-funktionellen Polysiloxans (α,ωN = 60)

In einem 500 mL Dreihalskolben wurden 10 g 1,1,3,3-Tetramethyl-disiloxan, 335 g Decamethylpentasiloxan, 0,35 g eines sauren Katalysators gemischt und 4 Stunden bei 80 °C gerührt. Nach dem Abkühlen wurden 6 g NaHCO₃ zugegeben und bei Raumtemperatur 12 Stunden gerührt. Nach einer Filtration wurde ein klares Produkt mit einem SiH-Wert von 0,04 % erhalten.

### b) Herstellung eines Epoxysiloxans (α,ω N = 60)

In einem 500 mL Dreihalskolben wurden 250 g der gemäß 2a hergestellten Verbindung und 8 g Allylglycidylether zusammen vorgelegt und auf 100 °C aufgeheizt. Anschließend wurden 15 ppm eines Platin-Katalysators zugesetzt und drei Stunden lang gerührt. Anschließend wurde das Reaktionsgemisch im Ölpumpenvakkum bei 120 °C zwei Stunden lang von flüchtigen Bestandteilen befreit. Man erhielt ein klares Produkt mit einem Epoxywert von 0,65 %.

### c) Umsetzung zum quaternären Polysiloxan-Polymer (α,ω N = 60)

In einem 500 mL Dreihalskolben wurden 27 g 3-N,N-Di-methyl-aminopropyl-laurylsäureamid, 5,5 g Essigsäure und 130 g Isopropanol bei Raumtemperatur gerührt. Anschließend wurden 200 g der gemäß 2b hergestellten Verbindung zugetropft. Anschließend wurde 8 Stunden bei 50 °C gerührt und schließlich wurden die flüchtigen Anteile im Ölpumpenvakkum bei 100 °C aus dem Reaktionsgemisch entfernt. Es wurde eine trübe hochviskose Flüssigkeit erhalten, welches durch die folgende statistische Formel beschrieben wird: 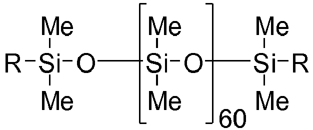 R =

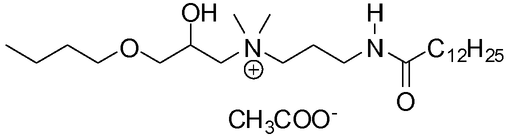

### Beispiel 3: Anwendungstechnische Eigenschaften der kationischen Polysiloxane mit quaternären Funktionen

Herstellung und Überprüfung von Haarbehandlungsmitteln unter Verwendung der Verbindungen Beispiel 1 und Beispiel 2.

Für die anwendungstechnische Beurteilung wurden Haartressen, die durch eine Bleichbehandlung standardisiert vorgeschädigt wurden, verwendet. Dazu werden friseurübliche Produkte verwendet. Die Schädigung der Haartressen wird im Detail in der DE 103 27 871 beschrieben.

Testrezepturen und Vorbehandlung der Haartressen:
Für die anwendungstechnische Beurteilung wurden die Verbindungen Beispiel 1 und Beispiel 2 in einer einfachen kosmetischen Formulierung eingesetzt.

Die Anwendungseigenschaften beim Einsatz in Haarspülungen wurden in den folgenden Rezepturen überprüft (Tab. 1):

**Tab.1: Haarspülung Formulierungen zur Austestung der haarkonditionierenden Eigenschaften.**

| **Formulierungs-Beispiele** | **0a** | **1a** | **2a** |
|---|---|---|---|
| TEGINACID® C, Evonik Goldschmidt GmbH (INCI: Ceteareth-25) | 0,5 % | 0,5 % | 0,5 % |
| TEGO® Alkanol 16, Evonik Goldschmidt GmbH (INCI: Cetyl Alcohol)) | 4 % | 4 % | 4 % |
| VARISOFT® 300, 30 %-ig, Evonik Goldschmidt GmbH (INCI: Cetrimonium Chloride) | 3,3 % | 3,3 % | 3,3 % |
| Wasser, demineralisiert | ad 100.0 | | |
| Zitronensäure | ad. pH 4,0 ± 0,3 | | |
| Verbindung Beispiel 1 | - | 0,5 % | - |
| Verbindung Beispiel 2 | - | - | 0,5 % |

Die Zusammensetzung der Testformulierungen ist zur Vermeidung der Beeinflussung der Testergebnisse durch (normalerweise vorhandene) Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten. Insbesondere die Kombination mit weiteren Inhaltsstoffen kann bei den beschriebenen Effekten zu einer synergistischen Verbesserung führen.

Die Vorbehandlung der Haare erfolgt durch eine Shampooformulierung (Tab. 2), welches keine Konditioniermittel enthält.

**Tab. 2: Shampooformulierung für die Vorbehandlung der Haartressen.**

| | |
|---|---|
| Texapon NSO®, 28 %-ig, Cognis (INCI: Sodium Laureth Sulfate) | 42,9 % |
| NaCl | 3 % |
| Wasser, demineralisiert | ad 100,0 |

Standardisierte Behandlung von vorgeschädigten Haarsträhnen mit konditionierenden Proben:
Die, wie oben beschrieben, vorgeschädigten Haarsträhnchen werden mit der Shampooformulierung Tab. 2 gewaschen. Hierbei werden die Haarsträhnen unter fließendem, warmem Wasser benetzt. Das überschüssige Wasser wird leicht von Hand ausgedrückt, dann wird das Shampoo aufgebracht und 1min lang sanft im Haar eingearbeitet (0.5 ml/2 g Haarsträhne). Die Haartresse wird für 30s unter fließend warmen Wasser ausgespült. Diese Prozedur wird ein weiteres mal wiederholt, nur dass abschließend 1 min lang ausgespült wird.

Anschließend werden direkt nach dem Waschen die Haarsträhnchen mit den Haarspülungs-Formulierungen Tab.1 konditioniert.
Hierbei wird die Spülung aufgebracht und sanft im Haar eingearbeitet (0.5 ml/2 g Haarsträhne). Nach einer Verweilzeit von 1 min wird das Haar für 1 min gespült.

Anschließend werden die mit den Haarspülungs-Formulierungen konditionierten Haarsträhnchen, vor der Schädigung der Haartressen mit dem Glätteisen und den anschließenden DSC Messungen (Differntial Scanning Calorimetry), an der Luft bei 50 % Luftfeuchtigkeit und 25 °C für mindestens 12 h getrocknet.

### Schädigung der Haartressen:

Für die Hitzeschädigung der Haartressen wurde ein Babyliss Pro Tourmaline Pulse Glätteisen verwendet.
Vor der Hitzebehandlung der Haartressen, wurde das Glätteisen auf der höchsten Stufe (Stufe 5, entspricht ca einer Temperatur von 190 °C) vorgeheizt.

Anschließend wurden die Haartressen dreimal für jeweils 10 Sekunden mit dem Glätteisen behandelt.

Bestimmung der Haarschädigung durch Hitzebehandlung mit Glätteisen:
Die mit dem Glätteisen geschädigten Haare wurden daraufhin mit Differential Scanning Calorimetry vermessen, um den Schädigungsgrad der Haare zu bestimmen. Bei der Differential Scanning Calorimetry handelt es sich um eine Standardmethode zur Bestimmung des Schädigungsgrades von Haaren. Details werden in dem Artikel von F.J. Wortmann "Investigations of cosmetically treated human hair by differential scanning calorimetry in water" (J. Cosmet. Sci., 53, 219-228, 2002) beschrieben. Bei dieser Meßmethode werden bei zunehmender Haarschädigung niedrigere Temperaturen ermittelt.

Als Referenzwert wurde eine mit Formulierung 0a behandelte Haartresse, die nicht mit dem Glätteisen behandelt wurde mit Differential Scanning Calorimetry (DSC) vermessen.

### DSC Messergebnisse:

In Figur 1 sind die erhaltenen DSC Messergebnisse zusammengefasst. Die Referenz (Haartresse mit Formulierung 0a konditioniert, keine anschließende Hitzebehandlung) hat die höchste Denaturierungstemperatur und daher die geringste Schädigung des Haares. Die Haartresse, die mit Formulierung 0a konditioniert und anschließend mit dem Glätteisen behandelt wurde, hat die niedrigste Denaturierungstemperatur und es liegt die stärkste Schädigung des Haares vor. Bei Verwendung der Verbindungen Beispiele 1 und Beispiel 2 in den Formulierungen 1a und 2a werden signifikant höhere Denaturierungstemperaturen gemessen. Folglich kann die Schädigung des Haares durch die Verwendung von den Verbindungen Beispiel 1 und 2 reduziert werden. Der Effekt ist bei der Verwendung von Beispiel 1 ausgeprägter als bei der Verwendung von Beispiel 2.

### Formulierungsbeispiele:

Diese Formulierungsbeispiele zeigen, dass Polysiloxane mit endständigen quaternären Ammoniumgruppen in einer Vielzahl kosmetischer Formulierungen zum Schutz des Haares vor Hitzeschädigungen eingesetzt werden können.

**Formulierungsbeispiel 1) Clear Shampoo**

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,5% |
| Water | 57,5% |
| TEGO® Betain F 50, Evonik Goldschmidt GmbH, 38%-ig (INCI: Cocamidopropyl Betaine) | 8,0% |
| ANTIL® 171 Evonik Goldschmidt GmbH (INCI: PEG-18 Glyceryl Oleate/Cocoate) | 1,0% |
| NaCl | 0,0% |
| Preservative | q.s. |

**Formulierungsbeispiel 2) Clear Conditioning Shampoo**

| | |
|---|---|
| TEXAPON® NSO, Cognis, 28%-ig (INCI: Sodium Laureth Sulfate) | 32,0% |
| Verbindung Beispiel 1 | 1,0% |
| Perfume | 0,5% |
| Water | 57,5 |
| TEGO® Cosmo C 100, Evonik Goldschmidt GmbH, (INCI: Creatine) | 1,0% |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 3) Clear Conditioning Shampoo**

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| Verbindung Beispiel 1 | 1,00% |
| Perfume | 0,25% |
| Water | 56,25 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00% |
| NaCl | 0,30% |
| Preservative | q.s. |

**Formulierungsbeispiel 4) Clear Conditioning Shampoo**

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| ABIL® Quat 3272, Evonik Goldschmidt GmbH (INCI: Quaternium-80) | 0,75% |
| Verbindung Beispiel 1 | 0,50% |
| Perfume | 0,25% |
| Water | 56,00 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00% |
| NaCl | 0,30% |
| Preservative | q.s. |

**Formulierungsbeispiel 5) Clear Conditioning Shampoo**

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| ABIL® B 8832, Evonik Goldschmidt GmbH (INCI: Bis-PEG/PPG-20/20 Dimethicone) | 1,00% |
| Verbindung Beispiel 1 | 0,50% |
| Perfume | 0,25% |
| Water | 55,75 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00% |
| NaCl | 0,30% |
| Preservative | q.s. |

**Formulierungsbeispiel 6) Clear Conditioning Shampoo**

| | |
|---|---|
| TEXAPON® NSO, Cognis, 28%-ig (INCI: Sodium Laureth Sulfate) | 32,00% |
| VARISOFT® PATC, Evonik Goldschmidt GmbH(INCI: Palmitamidopropyltrimonium Chloride) | 1,50% |
| REWODERM® LI S 80, Evonik Goldschmidt GmbH (INCI: PEG-200 Hydrogenated Glyceryl Palmate; PEG-7 Glyceryl Cocoate) | 2,00% |
| Verbindung Beispiel 1 | 0,50% |
| Perfume | 0,25% |
| Water | 54,05 |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 7) Clear Conditioning Shampoo**

| | |
|---|---|
| TEXAPON® NSO, Cognis, 28%-ig (INCI: Sodium Laureth Sulfate) | 32,00% |
| REWODERM® LI S 80, Evonik Goldschmidt GmbH (INCI: PEG-200 Hydrogenated Glyceryl Palmate; PEG-7 Glyceryl Cocoate) | 2,00% |
| Verbindung Beispiel 1 | 0,50% |
| Perfume | 0,25% |
| Water | 55,55 |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 8) Pearlized Shampoo**

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,25% |
| Water | 55,25 |
| TEGO® Betain F 50, Evonik Goldschmidt GmbH, 38%-ig (INCI: Cocamidopropyl Betaine) | 8,00% |
| TEGO® Pearl N 300 Evonik Goldschmidt GmbH (INCI: Glycol Distearate; Laureth-4; Cocamidopropyl Betaine) | 2,00% |
| ANTIL® 171 Evonik Goldschmidt GmbH (INCI: PEG-18 Glyceryl Oleate/Cocoate) | 1,50% |
| NaCl | 0,50% |
| Preservative | q.s. |

**Formulierungsbeispiel 9) 2 in 1 Shampoo**

| | |
|---|---|
| TEXAPON® NSO, Cognis, 28%-ig (INCI: Sodium Laureth Sulfate) | 32,00% |
| VARISOFT® PATC, Evonik Goldschmidt GmbH(INCI: Palmitamidopropyltrimonium Chloride) | 1,50% |
| REWODERM® LI S 80, Evonik Goldschmidt GmbH (INCI: PEG-200 Hydrogenated Glyceryl Palmate; PEG-7 Glyceryl Cocoate) | 2,00% |
| Verbindung Beispiel 1 | 0,50% |
| Perfume | 0,25% |
| Water | 54,05 |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 10) Rinse-Off Conditioner**

| | |
|---|---|
| Water | 90,50% |
| VARISOFT® BT 85, Evonik Goldschmidt GmbH (INCI: Behentrimonium Chloride) | 3,00% |
| Verbindung Beispiel 1 | 1,50% |
| TEGO® Alkanol 1618, Evonik Goldschmidt GmbH (INCI: Cetearyl Alcohol) | 5,00 |
| Preservative, Perfume | q.s. |

**Formulierungsbeispiel 11) Rinse-Off Conditioner**

| | |
|---|---|
| Water | 90,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| Verbindung Beispiel 1 | 0,80% |
| TEGO® Alkanol 1618, Evonik Goldschmidt GmbH (INCI: Cetearyl Alcohol) | 5,00 |
| Preservative, Perfume | q.s. |

**Formulierungsbeispiel 12) Rinse-Off Conditioner**

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® Quat 3272, Evonik Goldschmidt GmbH (INCI: Quaternium-80) | 1,00% |
| Verbindung Beispiel 1 | 0,80% |
| TEGO® Alkanol 1618, Evonik Goldschmidt GmbH (INCI: Cetearyl Alcohol) | 5,00 |
| Preservative, Perfume | q.s. |

**Formulierungsbeispiel 13) Rinse-Off Conditioner**

| | |
|---|---|
| TEGINACID® C, Evonik Goldschmidt GmbH (INCI: Ceteareth-25) | 0,50% |
| TEGO® Alkanol 16, Evonik Goldschmidt GmbH (INCI: Cetyl Alcohol) | 2,00% |
| TEGO® Amid S 18, Evonik Goldschmidt GmbH (INCI: Stearamidopropyl Dimethylamine) | 1,00% |
| Verbindung Beispiel 1 | 1,50% |
| Propylene Glycol | 2,00% |
| Citric Acid Monohydrate | 0,30% |
| Water | 92,70% |
| Preservative, Perfume | q.s. |

**Formulierungsbeispiel 14) Rinse-Off Conditioner**

| | |
|---|---|
| TEGINACID® C, Evonik Goldschmidt GmbH (INCI: Ceteareth-25) | 0,50% |
| TEGO® Alkanol 16, Evonik Goldschmidt GmbH (INCI: Cetyl Alcohol) | 5,00% |
| TEGOSOFT® DEC, Evonik Goldschmidt GmbH (INCI: Diethylhexyl Carbonate) | 1,00% |
| Verbindung Beispiel 1 | 1,50% |
| Water | 89,20% |
| TEGO® Cosmo C 100 Evonik Goldschmidt GmbH (INCI: Creatine) | 0,50% |
| Propylene Glycol | 2,00% |
| Citric Acid Monohydrate | 0,30% |
| Preservative, Perfume | q.s. |

**Formulierungsbeispiel 15) Leave-In Conditioner Spray**

| | |
|---|---|
| Lactic Acid, 80% | 0,40% |
| Water | 95,30% |
| TEGO® Amid S 18, Evonik Goldschmidt GmbH (INCI: Stearamidopropyl Dimethylamine) | 1,20% |
| TEGIN® G 1100 Pellets, Evonik Goldschmidt GmbH (INCI: Glycol Distearate) | 0,60% |
| TEGO® Care PS, Evonik Goldschmidt GmbH (INCI: Methyl Glucose Sesquistearate) | 1,20% |
| TEGOSOFT® DEC, Evonik Goldschmidt GmbH (INCI: Diethylhexyl Carbonate) | 0,30% |
| Verbindung Beispiel 1 | 1,00% |
| Preservative, Perfume | q.s. |

**Formulierungsbeispiel 16) Leave-In Conditioner Spray**

| | |
|---|---|
| TAGAT® CH-40, Evonik Goldschmidt GmbH (INCI: PEG-40 Hydrogenated Castor Oil) | 2,00% |
| Ceramide VI, Evonik Goldschmidt GmbH (INCI: Ceramide 6 II) | 0,05% |
| Perfume | 0,20% |
| Water | 90,95% |
| Verbindung Beispiel 1 | 0,50% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 2,00% |
| TEGO® Betain F 50 Evonik Goldschmidt GmbH 38% (INCI: Cocamidopropyl Betaine) | 2,30% |
| Citric Acid (10% in water) | 2,00% |

**Formulierungsbeispiel 17) Leave-In Conditioner Foam**

| | |
|---|---|
| Verbindung Beispiel 1 | 0,50% |
| TAGAT® CH-40, Evonik Goldschmidt GmbH (INCI: PEG-40 Hydrogenated Castor Oil) | 0,50% |
| Perfume | 0,30% |
| TEGO® Betain 810, Evonik Goldschmidt GmbH (INCI: Capryl/Capramidopropyl Betaine) | 2,00% |
| Water | 94,00% |
| TEGO® Cosmo C 100, Evonik Goldschmidt GmbH (INCI: Creatine) | 0,50% |
| TEGOCEL® HPM 50, Evonik Goldschmidt GmbH (INCI: Hydroxypropyl Methylcellulose) | 0,30% |
| VARISOFT® 300, Evonik Goldschmidt GmbH (INCI: Cetrimonium Chloride) | 1,30% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 0,50% |
| Citric Acid, 30% | 0,10% |
| Preservative | q.s. |

**Formulierungsbeispiel 18) Strong Hold Styling Gel**

| | |
|---|---|
| TEGO® Carbomer 141, Evonik Goldschmidt GmbH (INCI: Carbomer) | 1,20% |
| Water | 67,00% |
| NaOH, 25% | 2,70% |
| PVP/VA W-735, ISP (INCI: PVP/VA Copolymer | 16,00% |
| Verbindung Beispiel 1 | 0,50% |
| Alcohol Denat. | 10,30% |
| TAGAT® O 2 V, Evonik Goldschmidt GmbH (INCI: PEG-20 Glyceryl Oleate) | 2,00% |
| Perfume | 0,30% |
| ABIL® B 88183, Evonik Goldschmidt GmbH (INCI: PEG/PPG-20/6 Dimethicone) | 0,30% |
| Preservative | q.s. |

**Formulierungsbeispiel 19) Schaumiges Körperpflegemittel**

| | |
|---|---|
| TEXAPON® NSO, Cognis, 28%-ig (INCI: Sodium Laureth Sulfate) | 14,30% |
| Perfume | 0,30% |
| Verbindung Beispiel 1 | 0,50% |
| REWOTERIC® AM C, Evonik Goldschmidt GmbH, 32%-ig (INCI: Sodium Cocoamphoacetate) | 8,00% |
| Water | 74,90% |
| TEGOCEL® HPM 50, Evonik Goldschmidt GmbH (INCI: Hydroxypropyl Methylcellulose) | 0,50% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1,00% |
| Citric Acid Monohydrate | 0,50% |

**Formulierungsbeispiel 20) Körperpflegemittel**

| | |
|---|---|
| TEXAPON® NSO, Cognis, 28%-ig (INCI: Sodium Laureth Sulfate) | 30,00% |
| TEGOSOFT® PC 31, Evonik Goldschmidt GmbH (INCI: Polyglyceryl-3 Caprate) | 0,50% |
| Verbindung Beispiel 1 | 0,50% |
| Perfume | 0,30% |
| Water | 53,90% |
| TEGOCEL® HPM 4000, Evonik Goldschmidt GmbH (INCI: Hydroxypropyl Methylcellulose) | 0,30% |
| REWOTERIC® AM C, Evonik Goldschmidt GmbH, 32%-ig (INCI: Sodium Cocoamphoacetate) | 10,00% |
| Citric Acid Monohydrate | 0,50% |
| REWODERM® LI S 80, Evonik Goldschmidt GmbH (INCI: PEG-200 Hydrogenated Glyceryl Palmate; PEG-7 Glyceryl Cocoate) | 2,00% |
| TEGO® Pearl N 300, Evonik Goldschmidt GmbH (INCI: Glycol Distearate; Laureth-4; Cocamidopropyl Betaine) | 2,00% |

**Formulierungsbeispiel 21) Schaumiges Körperpflegemittel**

| | |
|---|---|
| TEXAPON® NSO, Cognis, 28%-ig (INCI: Sodium Laureth Sulfate) | 14,30% |
| Perfume | 0,30% |
| Verbindung Beispiel 1 | 0,50% |
| REWOTERIC® AM C, Evonik Goldschmidt GmbH, 32%-ig (INCI: Sodium Cocoamphoacetate) | 8,00% |
| Water | 75,10% |
| Polyquaternium-7 | 0,30 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1,00% |
| Citric Acid Monohydrate | 0,50% |

**Formulierungsbeispiel 22) Mild Foam Bath**

| | |
|---|---|
| TEXAPON® NSO, Cognis, 28%-ig (INCI: Sodium Laureth Sulfate) | 27,00% |
| REWOPOL® SB FA 30, Evonik Goldschmidt GmbH, 40%-ig (INCI: Disodium Laureth Sulfosuccinate) | 12,00% |
| TEGOSOFT® LSE 65 K SOFT, Evonik Goldschmidt GmbH (INCI: Sucrose Cocoate) | 2,00% |
| Water | 39,00% |
| REWOTERIC® AM C, Evonik Goldschmidt GmbH, 32%-ig (INCI: Sodium Cocoamphoacetate) | 13,00% |
| Verbindung Beispiel 1 | 0,50% |
| Citric Acid (30% in water) | 3,00% |
| ANTIL® 171 Evonik Goldschmidt GmbH (INCI: PEG-18 Glyceryl Oleate/Cocoate) | 1,50% |
| TEGO® Pearl N 300 Evonik Goldschmidt GmbH (INCI: Glycol Distearate; Laureth-4; Cocamidopropyl Betaine) | 2,00% |

**Formulierungsbeispiel 23) Schaumiges Körperpflegemittel**

| | |
|---|---|
| TEGOCEL® HPM 50, Evonik Goldschmidt GmbH (INCI: Hydroxypropyl Methylcellulose) | 0,50% |
| Water | 80,10% |
| Perfume | 0,20% |
| Verbindung Beispiel 1 | 0,50% |
| TEGOSOFT® GC, Evonik Goldschmidt GmbH, (INCI: PEG-7 Glyceryl Cocoate) | 1,30% |
| TEGO® Betain 810, Evonik Goldschmidt GmbH (INCI: Capryl/Capramidopropyl Betaine) | 16,90% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 0,50% |
| Preservative | q.s. |

**Formulierungsbeispiel 24) Clear Shampoo**

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,25% |
| Water | 56,05% |
| TEGO® Betain F 50, Evonik Goldschmidt GmbH, 38%-ig (INCI: Cocamidopropyl Betaine) | 8,00% |
| ANTIL® 171 Evonik Goldschmidt GmbH (INCI: PEG-18 Glyceryl Oleate/Cocoate) | 2,50% |
| NaCl | 0,70% |
| Preservative | q.s. |

**Formulierungsbeispiel 25) Clear Shampoo**

| | |
|---|---|
| TEXAPON® NSO, Cognis, 28%-ig (INCI: Sodium Laureth Sulfate) | 32,00% |
| Verbindung Beispiel 1 | 0,50% |
| Perfume | 0,25% |
| Water | 55,35% |
| REWOTERIC® AMC, Evonik Goldschmidt GmbH, (INCI: Sodium Cocoamphoacetate) | 9,40% |
| ANTIL® 171 Evonik Goldschmidt GmbH (INCI: PEG-18 Glyceryl Oleate/Cocoate) | 2,50% |
| Preservative | q.s. |

**Formulierungsbeispiel 25) Clear Shampoo**

| | |
|---|---|
| TEXAPON® NSO, Cognis, 28%-ig (INCI: Sodium Laureth Sulfate) | 17,90% |
| Verbindung Beispiel 1 | 0,50% |
| Perfume | 0,25% |
| Water | 62,50% |
| TEGO® Betain F 50, Evonik Goldschmidt GmbH, 38%-ig (INCI: Cocamidopropyl Betaine) | 6,60% |
| REWOPOL® SB FA 30, Evonik Goldschmidt GmbH, (INCI: Disodium Laureth Sulfosuccinate) | 6,25% |
| ANTIL® 171 Evonik Goldschmidt GmbH (INCI: PEG-18 Glyceryl Oleate/Cocoate) | 5,00% |
| NaCl | 1,00% |
| Preservative | q.s. |

**Formulierungsbeispiel 26) Rinse-Off Conditioner**

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® OSW 5, Evonik Goldschmidt GmbH (INCI: Cyclopentasiloxane; Dimethiconol) | 1,00% |
| Verbindung Beispiel 1 | 0,80% |
| TEGO® Alkanol 1618, Evonik Goldschmidt GmbH (INCI: Cetearyl Alcohol) | 5,00 |
| Preservative, Perfume | q.s. |

**Formulierungsbeispiel 27) Rinse-Off Conditioner**

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® Soft AF 100, Evonik Goldschmidt GmbH (INCI: Methoxy PEG/PPG-7/3 Aminopropyl Dimethicone) | 1,00% |
| Verbindung Beispiel 1 | 0,80% |
| TEGO® Alkanol 1618, Evonik Goldschmidt GmbH (INCI: Cetearyl Alcohol) | 5,00 |
| Preservative, Perfume | q.s. |

**Formulierungsbeispiel 28) Rinse-Off Conditioner**

| | |
|---|---|
| Water | 89,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| SF 1708, Momentive (INCI: Amodimethicone) | 1,00% |
| Verbindung Beispiel 1 | 0,80% |
| TEGO® Alkanol 1618, Evonik Goldschmidt GmbH (INCI: Cetearyl Alcohol) | 5,00 |
| Preservative, Perfume | q.s. |

**Formulierungsbeispiel 29) Conditioning Shampoo**

| | |
|---|---|
| TEXAPON® NSO, Cognis, 28%-ig (INCI: Sodium Laureth Sulfate) | 27,00% |
| Plantacare 818 UP, Cognis 51.4%-ig (INCI: Coco Glucoside) | 5,00% |
| T-Quat | 1,50% |
| Perfume | 0,25% |
| Water | 56,55 |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 30) Conditioning Shampoo**

| | |
|---|---|
| Plantacare 818 UP, Cognis 51.4%-ig (INCI: Coco Glucoside) | 18,00% |
| T-Quat | 1,50% |
| Perfume | 0,25% |
| Water | 70,55% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00% |
| NaCl | 0,50% |
| Preservative | q.s. |

## Patentansprüche

1. Verwendung von Polysiloxanen beinhaltend mindestens eine quaternäre Ammoniumgruppe zum Schutz von tierischen oder menschlichen Haaren vor Hitzeschädigung, **dadurch gekennzeichnet, dass** mindestens eine quaternäre Ammoniumgruppe endständig an das Polysiloxan gebunden ist,
wobei die quaternäre Ammoniumgruppe an das Siloxangerüst über eine M'-Einheit der allgemeinen Formel XSiY₂O_{1/2}
mit
X = gleiche oder verschiedene organische Reste, die quaternäre Ammoniumgruppen tragen,
Y = gleiche oder verschiedene Reste aus der Gruppe Alkyl, Aryl, oder Alkaryl mit 1 bis 30 C-Atomen,
gebunden ist.

2. Verwendung von Polysiloxanen gemäß Anspruch 1, **dadurch gekennzeichnet, dass** insgesamt genau zwei quaternäre Ammoniumgruppen enthalten sind und diese endständig an das Polysiloxan gebunden sind.

3. Verwendung von Polysiloxanen gemäß Anspruch 1 oder 2, **dadurch gekennzeichnet, dass** Polysiloxane gemäß der allgemeinen Formel I verwendet werden,
[M' Dₙ]₃ T Formel I
wobei
M' = XSiY₂O_{1/2},
D = SiY₂O_{2/2},
T = SiZO_{3/2},
X = gleiche oder verschiedene organische Reste, die quaternäre Ammoniumgruppen tragen,
Y = gleiche oder verschiedene Reste aus der Gruppe Alkyl, Aryl, oder Alkaryl mit 1 bis 30 C-Atomen,
Z = gleiche oder verschiedene Reste aus der Gruppe Alkyl, Aryl, oder Alkaryl mit 1 bis 30 C-Atomen,
n = 2 bis 200
sind.

4. Verwendung von Polysiloxanen gemäß Anspruch 3, **dadurch gekennzeichnet, dass** das Polysiloxane gemäß der allgemeinen Formel I Reste X mit dem Aufbau -R1-R2 aufweist, wobei
R1 vorzugsweise gleiche oder verschiedene zweiwertige Reste sind, ausgewählt aus der Gruppe 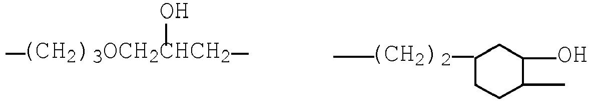 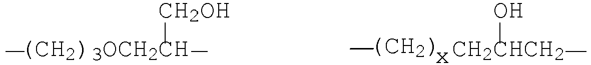 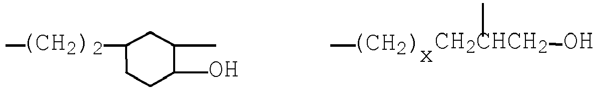 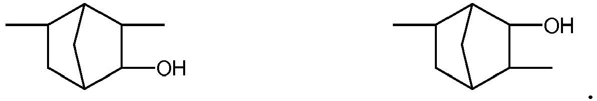 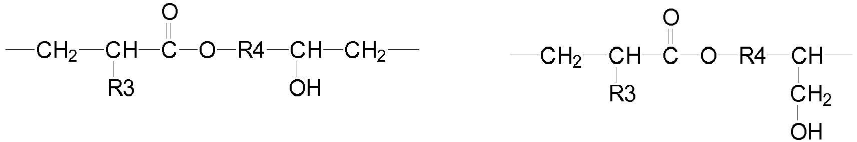
R2 ausgewählt ist aus der Gruppe bestehend aus 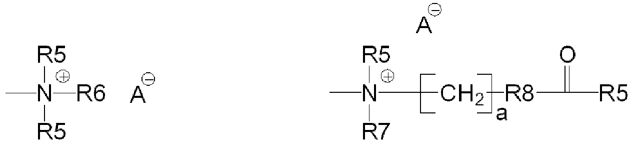 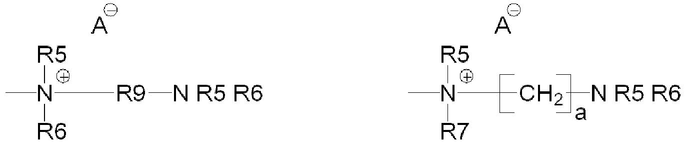 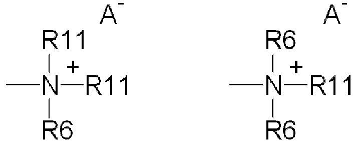
R3 gleiche oder verschiedene Reste aus der Gruppe Wasserstoff oder Alkyl mit 1 bis 6 C-Atomen sind,
R4 gleiche oder verschiedene zweiwertige Kohlenwasserstoffreste, die gegebenenfalls Etherfunktionen enthalten, sind,
R5, R6, R7 jeweils unabhängig voneinander Alkylreste mit 1 bis 30 C-Atomen sind,
R8 gleiche oder verschiedene Reste aus der Gruppe -O-; -NR10 sind,
R9 gleiche oder verschiedene gegebenenfalls verzweigte divalente Kohlenwasserstoffreste sind,
R10 gleiche oder verschiedene Reste aus der Gruppe Wasserstoff oder Alkyl mit 1 bis 6 C-Atomen sind,
R11 gleiche oder verschiedene Reste der allgemeinen Formel 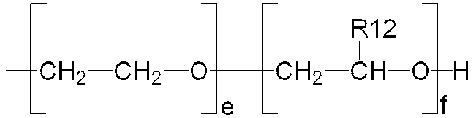 sind,
R12 gleiche oder verschiedene Alkyl-, Aryl- oder Alkarylreste mit 1 bis 30 C-Atomen, die gegebenenfalls Etherfunktionen enthalten, sind,
e 0 bis 20 ist
f 0 bis 20 ist,
e + f >= 1 ist
x 2 bis 18 ist,
a 2 bis 18 ist,
und
A⁻ gleiche oder verschiedene Gegenionen zu den positiven Ladungen an den quaternären Ammoniumgruppen, ausgewählt aus anorganischen oder organischen Anionen der Säuren HA sind.

5. Verwendung von Polysiloxanen gemäß mindestens einem der Ansprüche 1 bis 4, **dadurch gekennzeichnet, dass** das Polysiloxan in Form einer kosmetischen Formulierung appliziert wird.

6. Verwendung von Polysiloxanen gemäß Anspruch 5, **dadurch gekennzeichnet, dass** das Polysiloxan in Form einer reinigenden und pflegenden Formulierung appliziert wird.

7. Kosmetischen Formulierung zum Schutz von tierischen oder menschlichen Haaren vor Hitzeschädigung enthaltend ein Polysiloxan beinhaltend mindestens eine quaternäre Ammoniumgruppe wie in mindestens einem der Ansprüche 1 bis 4 beschrieben und einen UV-Lichtschutzfilter, **dadurch gekennzeichnet, dass** sie das Polysiloxan in einer Konzentration von 0.01 bis 20 Massenprozent beinhaltet.

## Claims

1. Use of polysiloxanes containing at least one quaternary ammonium group for protecting animal or human hair against heat damage, **characterized in that** at least one quaternary ammonium group is terminally bonded to the polysiloxane,
the quaternary ammonium group being bonded to the siloxane backbone via an M' unit of the general formula XSiY₂O_{1/2} where
X = identical or different organic radicals which carry quaternary ammonium groups,
Y = identical or different radicals from the group alkyl, aryl or alkaryl having 1 to 30 carbon atoms.

2. Use of polysiloxanes according to Claim 1, **characterized in that** overall exactly two quaternary ammonium groups are present and these are terminally bonded to the polysiloxane.

3. Use of polysiloxanes according to Claim 1 or 2, **characterized in that** polysiloxanes according to the general formula I are used,
[M' Dₙ]₃ T Formula I
where
M' = XSiY₂O_{1/2},
D = SiY₂O_{2/2},
T = SiZO_{3/2},
X = identical or different organic radicals which carry quaternary ammonium groups,
Y = identical or different radicals from the group alkyl, aryl, or alkaryl having 1 to 30 carbon atoms,
Z = identical or different radicals from the group alkyl, aryl or alkaryl having 1 to 30 carbon atoms,
n = 2 to 200.

4. Use of polysiloxanes according to Claim 3, **characterized in that** the polysiloxane according to the general formula I has radicals X with the structure -R1-R2, where
R1 are preferably identical or different divalent radicals selected from the group 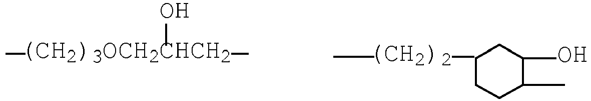 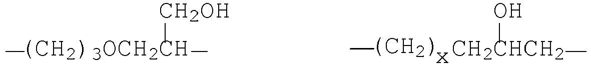 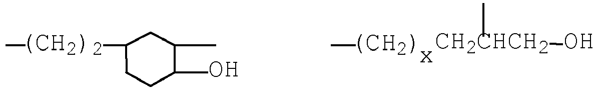 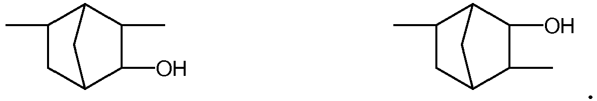 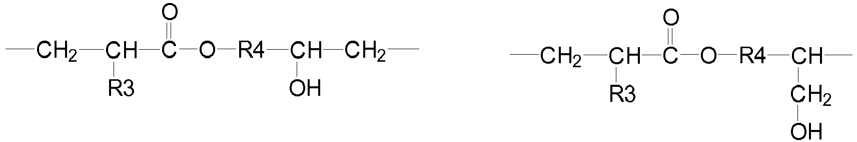
R2 is selected from the group consisting of 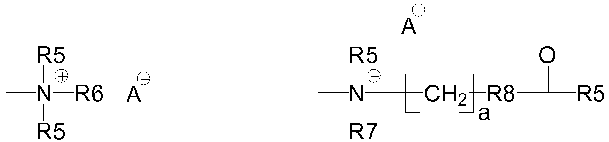 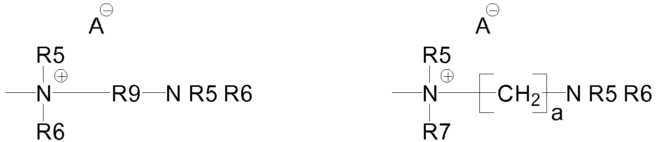 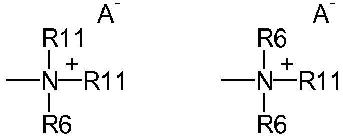
R3 are identical or different radicals from the group hydrogen or alkyl having 1 to 6 carbon atoms,
R4 are identical or different divalent hydrocarbon radicals which optionally comprise ether functions,
R5, R6, R7, in each case independently of one another, are alkyl radicals having 1 to 30 carbon atoms,
R8 are identical or different radicals from the group -O-; -NR10,
R9 are identical or different optionally branched divalent hydrocarbon radicals,
R10 are identical or different radicals from the group hydrogen or alkyl having 1 to 6 carbon atoms,
R11 are identical or different radicals of the general formula 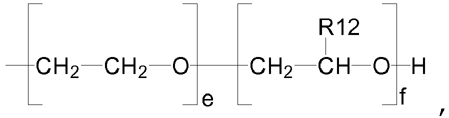
R12 are identical or different alkyl, aryl, or alkaryl radicals having 1 to 30 carbon atoms which optionally comprise ether functions,
e is 0 to 20
f is 0 to 20,
e + f is >= 1
x is 2 to 18,
a is 2 to 18,
and
A⁻ are identical or different counterions to the positive charges on the quaternary ammonium groups, selected from inorganic or organic anions of the acids HA.

5. Use of polysiloxanes according to at least one of Claims 1 to 4, **characterized in that** the polysiloxane is applied in the form of a cosmetic formulation.

6. Use of polysiloxanes according to Claim 5, **characterized in that** the polysiloxane is applied in the form of a cleaning and care formulation.

7. Cosmetic formulation for protecting animal or human hair against heat damage comprising a polysiloxane containing at least one quaternary ammonium group as described in at least one of Claims 1 to 4 and a UV light protection filter, **characterized in that** it contains the polysiloxane in a concentration of from 0.01 to 20% by mass.

## Revendications

1. Utilisation de polysiloxanes contenant au moins un groupe ammonium quaternaire pour la protection de poils animaux ou humains contre la dégradation thermique, **caractérisée en ce qu'**au moins un groupe ammonium quaternaire est lié en position terminale au polysiloxane, le groupe ammonium quaternaire étant lié à la structure siloxane via une unité M' de formule générale XSiY₂O_{1/2} dans laquelle
X = des radicaux organiques identiques ou différents, qui portent des groupes ammonium quaternaire,
Y = des radicaux identiques ou différents du groupe formé par alkyle, aryle ou alkaryle comprenant 1 à 30 atomes de carbone.

2. Utilisation de polysiloxanes selon la revendication 1, **caractérisée en ce qu'**ils contiennent au total exactement deux groupes ammonium quaternaire et ceux-ci sont liés en position terminale au polysiloxane.

3. Utilisation de polysiloxanes selon la revendication 1 ou 2, **caractérisée en ce qu'**on utilise des polysiloxanes selon la formule générale I,
[M' Dn]₃T Formule I
dans laquelle
M' = XSiY₂O_{1/2},
D = SiY₂O_{2/2},
T = SiZO_{3/2},
X = des radicaux organiques identiques ou différents, qui portent des groupes ammonium quaternaire,
Y = des radicaux identiques ou différents du groupe formé par alkyle, aryle ou alkaryle comprenant 1 à 30 atomes de carbone,
Z = des radicaux identiques ou différents du groupe formé par alkyle, aryle ou alkaryle comprenant 1 à 30 atomes de carbone,
n = 2 à 200.

4. Utilisation de polysiloxanes selon la revendication 3, **caractérisée en ce que** le polysiloxane selon la formule générale I présente des radicaux X de structure -R1-R2, dans laquelle
R1 représente de préférence des radicaux bivalents, identiques ou différents, choisis dans le groupe 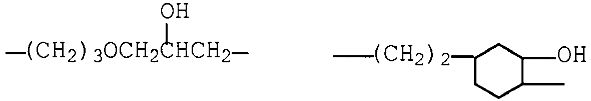 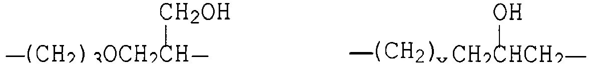 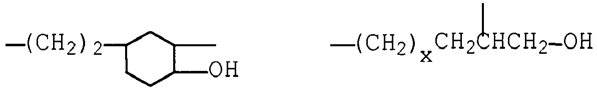 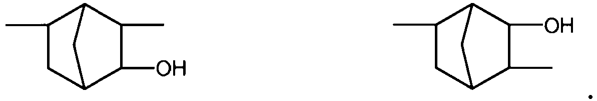 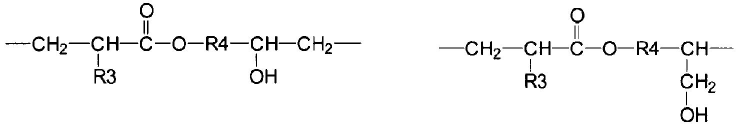
R2 est choisi dans le groupe constitué par : 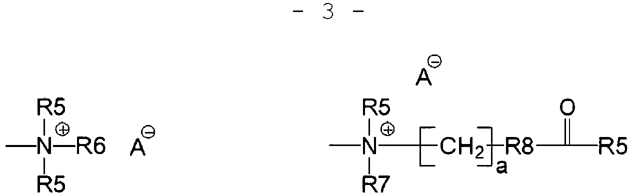 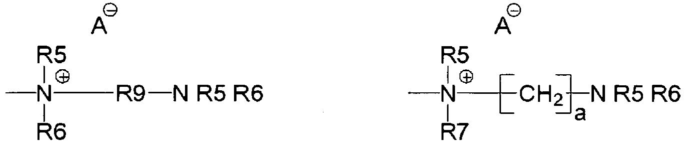 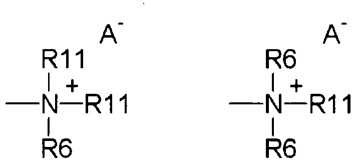
R3 représente des radicaux identiques ou différents du groupe formé par hydrogène ou alkyle comprenant 1 à 6 atomes de carbone,
R4 représente des radicaux hydrocarbonés bivalents, identiques ou différents, qui contiennent le cas échéant des fonctions éther,
R5, R6, R7 représentent, à chaque fois indépendamment l'un de l'autre, des radicaux alkyle comprenant 1 à 30 atomes de carbone,
R8 représente des radicaux identiques ou différents du groupe formé par -O- ; -NR10 ;
R9 représente des radicaux hydrocarbonés bivalents, identiques ou différents, le cas échéant ramifiés,
R10 représente des radicaux identiques ou différents du groupe formé par hydrogène ou alkyle comprenant 1 à 6 atomes de carbone,
R11 représente des radicaux identiques ou différents de formule générale 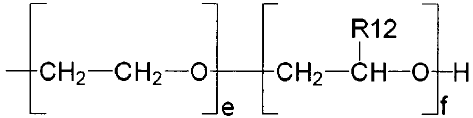
R12 représente des radicaux alkyle, aryle ou alkaryle identiques ou différents comprenant 1 à 30 atomes de carbone, qui contiennent le cas échéant des fonctions éther,
e vaut 0 à 20,
f vaut 0 à 20,
e + f est ≥ 1
x vaut 2 à 18,
a vaut 2 à 18 et
A⁻ représente des contre-ions, identiques ou différents, des charges positives sur les groupes ammonium quaternaire, choisis parmi les anions inorganiques ou organiques des acides HA.

5. Utilisation de polysiloxanes selon au moins l'une quelconque des revendications 1 à 4, **caractérisée en ce que** le polysiloxane est appliqué sous forme d'une formulation cosmétique.

6. Utilisation de polysiloxanes selon la revendication 5, **caractérisée en ce que** le polysiloxane est appliqué sous forme d'une formulation nettoyante et de soin.

7. Formulation cosmétique pour la protection de poils animaux ou humains contre la dégradation thermique contenant un polysiloxane comprenant au moins un groupe ammonium quaternaire tel que décrit dans au moins l'une quelconque des revendications 1 à 4 et un filtre de protection contre la lumière UV, **caractérisée en ce qu'**elle contient le polysiloxane en une concentration de 0,01 à 20% en masse.
